# EUROPÄISCHE PATENTANMELDUNG

(11) **EP 1 442 733 A1**
(43) Veröffentlichungstag der Anmeldung: **04.08.2004**
(21) Anmeldenummer: 03002286.7
(22) Anmeldetag: 03.02.2003
(51) Int. Cl.: A61F 13/15

(54) **Handhabungshilfe für Hygieneartikel**

(71) Anmelder: Rechsteiner, Urs, 8055 Zürich (CH)
(72) Erfinder: Kolly, Linda, 6212 St.Erhard (CH)

(57) **Zusammenfassung**

Beschrieben wird ein Hygieneartikel wie Damenbinde, Slipeinlage, Inkontinenzhilfe und dergleichen, mindestens dadurch gekennzeichnet, dass ein Faden (15) so angebracht ist, dass der Hygieneartikel nach Gebrauch durch einfache Handhabung ohne direkten Kontakt gerollt werden kann.

## Beschreibung

### Technisches Gebiet

Die Erfindung gehört dem Gebiet der Hygieneartikel an. Insbesondere betrifft die vorliegende Erfindung die Anbringung eines Fadens oder dergleichen am Hygieneartikel, der es ermöglicht, den Hygieneartikel nach Gebrauch zusammenzurollen und so für die Entsorgung vorzubereiten, ohne dabei direkten Kontakt zum Hygieneartikel selbst zu haben.

### Hintergrund der Erfindung

Hygieneartikel, welche der Absorbtion von Körperfluiden dienen, sind selbstverständlich allgemein bekannt. Grundsätzlich sind sie so aufgebaut, dass ein absorbierendes Element oder ein absorbierender Kern zwischen einem flüssigkeitsdurchlässigen körperberührenden Element und einem flüssigkeitsundurchlässigen Element dazwischengelegt ist. Austretende Körperfluide gelangen durch das flüssigkeitsdurchlässige Element in den absorbierenden Kern und werden dort aufgenommen respektive gebunden. Das flüssigkeitsundurchlässige Element dient dazu, dass die aus dem Kern austretenden oder davon ausgestossenen Körperfluide nicht durch den Hygieneartikel nach aussen dringen. flüssigkeitsundurchlässigen Elementes oder mit sogenannten Flügeln, angebracht auf der Seite des Hygieneartikels, versehen.

Herkömmliche Hygieneartikel werden nach dem Gebrauch durch die Benutzerin entsorgt, wobei sich ein direkter Kontakt mit dem Hygieneartikel bislang kaum vermeiden liess. Der direkte Kontakt mit dem Hygieneartikel ist aber nicht gewünscht, vor allem an Orten unsicherer Hygieneverhältnisse, auswärts und wenn der Hygieneartikel durch Dritte entsorgt wird (Spitäler, Altersheime, Geriatrien, etc.)

Davon ausgehend liegt der Erfindung die Aufgabe zugrunde, eine Handhabungshilfe für die Vorbereitung der Entsorgung des Hygieneartikels zu schaffen, die den direkten Kontakt mit dem Hygieneartikel ausschliesst, beim Tragen zu keiner Behinderung führt und möglichst einfach in bestehende Produktionsvorgänge integriert werden kann.

### Beschreibung der Darstellungen

Durch die begleitenden Zeichnungen und Beschreibungen soll die Verständlichkeit der vorliegenden Erfindung erhöht werden.
- FIG. 1:: Ansicht von oben auf die feuchtigkeitsdurchlässige Seite eines herkömmlichen Hygieneartikels, wobei Teile herausgeschnitten sind, um den Aufbau aufzuzeigen.
- FIG. 2:: Querschnitt eines herkömmlichen Hygieneartikels entlang der Linie Y-Y gemäss FIG. 1
- FIG. 3:: Ansicht von oben auf die feuchtigkeitsdurchlässige Seite eines Hygieneartikels mit eingelassenem Faden.
- FIG. 4:: Querschnittansicht entlang (42) gemäss FIG. 3, wobei nur das Seitenteil (32) gemäss FIG. 2 des Hygieneartikels betrachtet wird. Der Faden ist in dieser Darstellung im Verbindungselement zwischen Unterseite und Oberseite angebracht.
- FIG. 5:: Ansicht wie FIG. 4. Der Faden ist auf der Oberseite mit einer zusätzlichen Abdeckung angebracht.
- FIG. 6:: Ansicht wie FIG. 4. Der Faden ist auf der Längsseite mit einer zusätzlichen Abdeckung angebracht.
- FIG. 7:: Querschnittansicht entlang der Linie (40) gemäss FIG. 3, wobei nur der Ausschnitt zwischen den Schnittlinien (41) und (42) betrachtet wird. Der Faden ist in die Struktur verwoben.
- FIG. 8:: Ansicht von oben auf den hinteren Teil des Hygieneartikels. Der Faden ist im hinteren Bereich an der Struktur des Hygieneartikels befestigt.
- FIG. 9:: Ansicht von oben auf den vorderen Teil des Hygieneartikels. Der Faden ist ausserhalb des Hygieneartikels nicht durchgehend. Abschluss bildet je ein Klebeelement zur Befestigung des Fadens.
- FIG. 10:: Ansicht von oben auf den vorderen Teil des Hygieneartikels. Der Faden ist ausserhalb des Hygieneartikels durchgehend. Abschluss bildet ein Klebeelement zur Befestigung des Fadens.
- FIG. 11:: Ansicht auf den Hygieneartikels, nachdem durch Zug am Faden ein Zusammenrollen bewirkt wurde. Die gerollte Form bleibt bestehen, da durch die äusseren Klebestreifen ein Verkleben erfolgt.

### Beschreibung der Erfindung

Der Begriff "Hygieneartikel" bezieht sich auf Artikel, welche am Körper getragen werden und dazu dienen, Körperfluide zu absorbieren und aufzunehmen. Es können dies Damenbinden, Slipeinlagen, Inkontinenzhilfen, Babywindeln und dergleichen sein. Insbesondere handelt es sich dabei um sogenannte "Einweg-Hygieneartikel", was bedeutet, dass diese Artikel nicht zum waschen oder zur Wiederverwertung in der ursprünglichen Form gedacht sind. Vorgesehen ist, sie nach einmaligem Gebrauch dem Abfall zuzuführen, zu recyceln oder zu kompostieren.

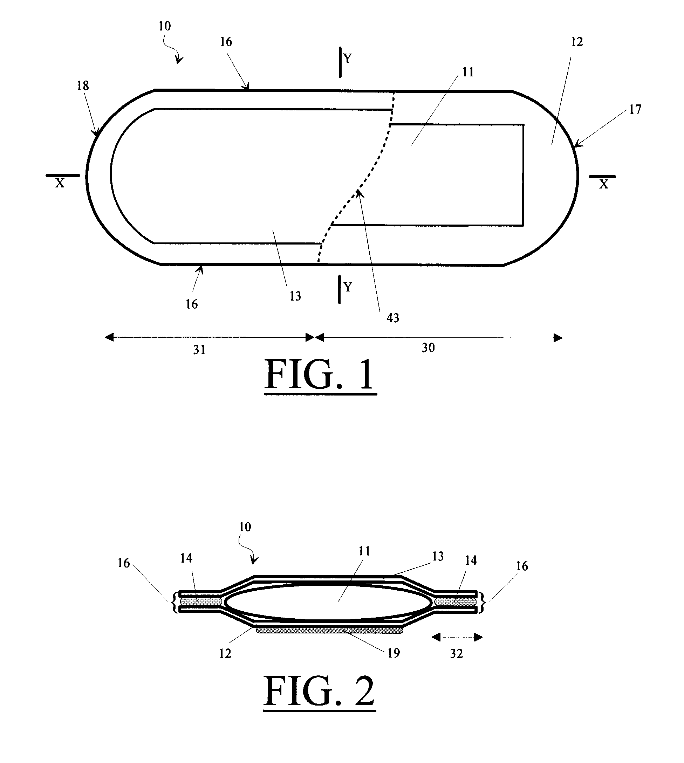

Ausgegangen wird von einem herkömmlichen Hygieneartikel wie in FIG. 1 dargestellt. Diese Hygieneartikel sind oft vorgeformt, vorgefaltet und aus hygienischen Gründen und Diskretionsansprüchen einzeln verpackt. Der Hygieneartikel wird abgegrenzt von zwei Längsseiten (16), einer Vorderseite (17), die der Vorderseite des Körpers zugewandt ist und einer Rückseite (18) die dem Rücken zugewandt ist.

In aller Regel sind die Hygieneartikel schichtweise aufgebaut und bestehen zumindest aus einer körperseitigen, flüssigkeitsdurchlässigen Abdeckung (Oberseite) (13), einer der Bekleidung zugewandten, flüssigkeitsundurchlässigen Abdeckung (Unterseite) (12) und einem zwischen diesen Schichten angeordneten absorbierenden Kern (11). Teilweise sind in den Hygieneartikeln weitere Schichten eingearbeitet.

Die Oberseite (13) steht im direkten Kontakt zum Körper und ist darauf ausgerichtet, Körperfluide aufzunehmen und durchfliessen zu lassen. Sie hat aus die Haut nicht irritierendem Material zu bestehen und sollte selbst möglichst keine Flüssigkeit absorbieren. Die Oberseite (13) wird hergestellt aus Wollstoff, synthetischen oder natürlichen Materialien oder Verbindungen derselben. Das Material soll dabei formbar sein, um den Tragkomfort zu erhöhen, gleichzeitig aber weitgehend resistent gegen übermässige Verformung oder gar ein Zerreissen während dem Tragen.

Die Unterseite (12) dient als Barriere zwischen dem absorbierenden Kern (11) und der Unterwäsche. Die Unterseite (12) wird gefertigt aus flüssigkeitsundurchlässigem oder -abweisendem Material. Gleichzeitig soll dieses Material möglichst atmungsaktiv und dampfdurchlässig sein. An der Unterseite angebracht ist in aller Regel eine Klebefläche (19) die entweder aus Streifen besteht oder durchgehend ist und die direkte Verbindung zur Unterwäsche sicherstellen soll.

Der Kern (11) ist zwischen Oberseite (13) und Unterseite (12) angebracht und nimmt über die Oberseite (13) Körperflüssigkeiten auf und absorbiert diese. Die dabei zur Anwendung gebrachten Materialien weisen eine hohe Absorbtionsfähigkeit auf. Es kommen Wolleflaum, Kunstseide, Baumwolle und verschiedene Polymere sowie Zusammensetzungen der genannten Materialien zur Anwendung. Die Grösse des Kerns (11) hängt von der beabsichtigten Verwendung des Einweg-Hygieneartikels ab.

Oberseite (13) und Unterseite (12) sind durch eine Befestigungsfläche (14) im Randbereich miteinander verbunden, wobei unterschiedliche Methoden der Verbindung zur Anwendung gelangen. Es können dies chemische, thermische oder mechanische Verbindungen zwischen der Oberseite (13) und der Unterseite (12) sein. In aller Regel liegt zwischen der Ober- (13) und der Unterseite (12) ein Drittmedium. Als Drittmedien kommen verschiedenste Klebstoffe oder -materialien und doppelseitig klebende Bänder zur Anwendung. Die Befestigungsfläche (14) kann durchgehend oder nur entlang der Längsseiten (16) ausgestaltet sein. Die Befestigungsfläche (14) zwischen Oberseite (13) und Unterseite (12) kann zudem unterschiedlich breit sein. Teilweise reicht sie bis an die Randbereiche.

In FIG. 1 ist der Einweg-Hygieneartikel so aufgeschnitten, dass der Aufbau des Einweg-Hygieneartikels erkennbar wird. Dieser Schnitt (43) unterteilt den Einweg-Hygieneartikel in einen aufgeschnittenen Bereich (30) und einen nicht aufgeschnittenen Bereich (31).

In grundsätzlicher Sicht ist die Erfindung so ausgestaltet, dass ein Faden (15) im Randbereich der Längsseiten (16) sowie der Rückseite (18) angebracht wird, der an der Vorderseite (17) aus der Struktur des Hygieneartikels austritt. Ein Zug an diesem Faden nach Gebrauch des Hygieneartikels führt zu einem Zusammenrollen des Hygieneartikels.

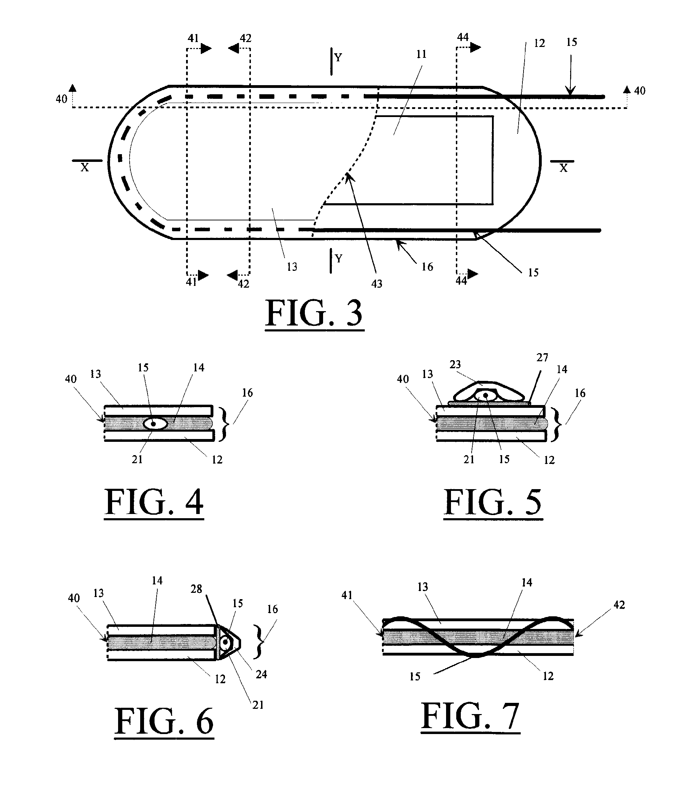

Bezugnehmend auf FIG. 3, 4, 5, 6, 7 wird der Faden (15) so in die Struktur herkömmlicher Hygieneartikel hineingearbeitet, dass er frei läuft. In FIG. 3 ist der Faden (15) im aufgeschnittenen Teil (30) des Hygieneartikels als durchgehende Linie dargestellt und im nicht aufgeschnittenen Bereich (31) als gestrichelte Linie. Um den Freilauf des Fadens zu gewährleisten kann dieser auch in einer Röhre (21) aus elastischem Material geführt werden. Diese Röhre (21) ist ihrerseits mit der Struktur des Hygieneartikels (10) direkt verbunden.

Gemäss FIG. 4, 5, 6, 7 kann der Faden (15) verschieden angebracht werden. FIG. 4 zeigt die Führung des Fadens (15) innerhalb der Befestigungsfläche (14). In FIG. 5 wurde der Faden (15) auf die Oberseite gelegt und mit einer zusätzlichen Abdeckung (23) und einem Klebeelement (27) befestigt. In FIG. 6 wird der Faden (15) entlang der Ränder des Hygieneartikels geführt und dort mit einer Abdeckung (24) und einem Klebeelement (28) befestigt. Fig. 7 zeigt die Längsansicht, wenn der Faden (15) durch mehrere Strukturen des Hygieneartikels (10) eingearbeitet ist (gestickt).

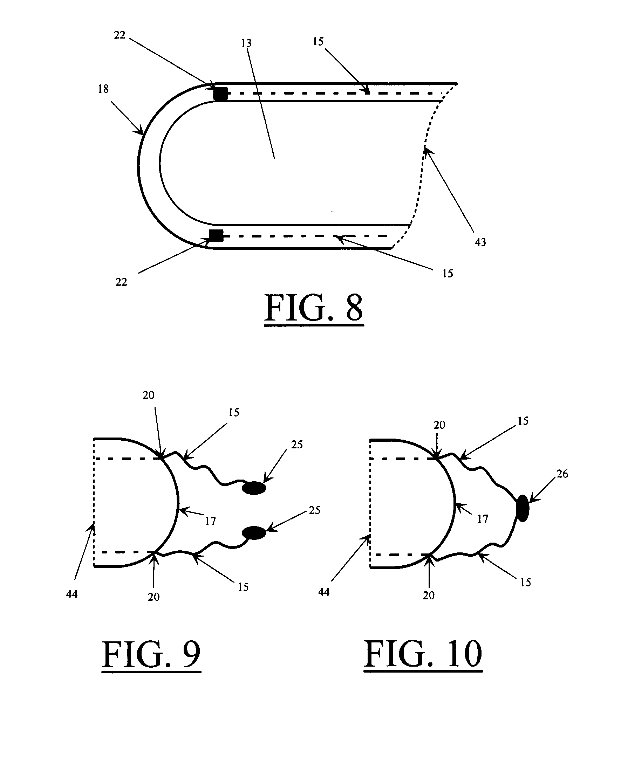

Der Faden (15) wird an der Rückseite (18) im Randbereich durchgehend geführt (FIG. 3). Beim Zug am Faden (15) soll ein fester Sitz desselben an der Rückseite (18) gewährleistet sein. Dazu wird der Faden (15) entweder in die Struktur eingearbeitet, mit dieser vernährt oder verschweisst. Der Faden (15) kann aber auch im hinteren Teil des Hygieneartikels mit Befestigungselementen (22) an der Struktur des Hygieneartikels befestigt werden (FIG. 9).

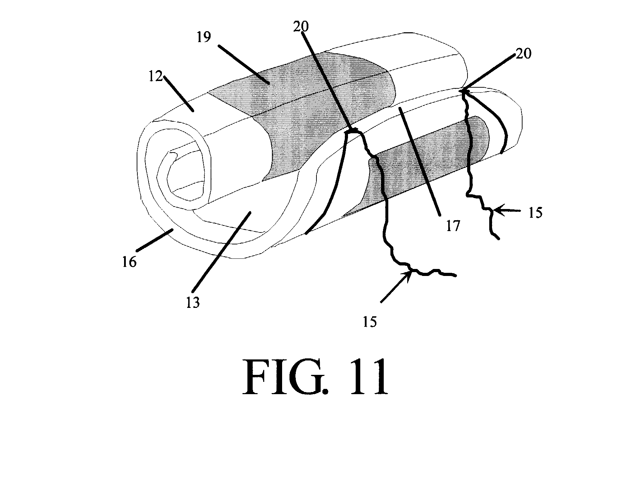

An der Vorderseite (17) tritt der Faden (15) an den Austrittspunkten (20) aus der Struktur des Hygieneartikels (10) aus. Sofern eine Röhre (21) verwendet wird, endet die Röhre beim Austritt aus der Struktur des Hygieneartikels (10). Die Austrittspunkte (20) sind durch Ösen oder ähnliche Elemente zu verstärken, um ein Aufreissen des Hygieneartikels beim Zug am Faden (15) zu verhindern. Der Faden (15) kann sowohl durchgehend sein (FIG. 11) als auch über zwei Enden verfügen (FIG. 10). Um den Faden (15) während dem Tragen am Hygieneartikel selbst oder an der Unterwäsche zu befestigen, kann ein Abschlussklebstreifen (25) (26) am Faden (15) angebracht werden.

Der Faden (15) wird gefertigt aus Baumwollfasern, Kunstseide, Polymeren sowie Zusammensetzungen der genannten Materialien sowie aus allen anderen Materialien, die hierfür aus dem Stand er Technik bekannt sind. Die Länge ist so zu bemessen, dass sie den gesamten Hygieneartikel umspannt und zudem genügend Spielraum für eine komfortable Handhabung lässt.

Nach Gebrauch des Hygieneartikels rollt sich der Hygieneartikel durch Zug am Faden selbständig auf und verklebt sich zudem im aufgerollten Zustand selbst durch die äusseren Klebstreifen (19). Weil der einzige Kontakt mit dem Hygieneartikel über den Faden (15) erfolgt, kommt es zu keinem direkten Kontakt mit dem Hygieneartikel. In der zusammengerollten Form kann der Hygieneartikel problemlos entsorgt werden.

## Patentansprüche

1. Handhabungshilfe für Hygieneartikel, wobei mindestens ein freilaufender Faden im Randbereich des Hygieneartikels zwischen der Unterseite (12) und der Oberseite (13) des Hygieneartikels angebracht ist, wobei der Faden an der Vorderseite (17) aus dem Hygieneartikel austritt und nach Gebrauch des Hygieneartikels durch Zug am Faden (15) ein Einrollen des Hygieneartikels bewirkt wird.

2. Hygieneartikel nach Anspruch 1, **dadurch gekennzeichnet, dass** der Faden auf der Oberseite (13) des Hygieneartikels, gehalten durch eine zusätzliche Abdeckung (23), angebracht ist.

3. Hygieneartikel nach Anspruch 1, **dadurch gekennzeichnet, dass** der Faden auf den Längsseiten (16) und der Rückseite (18) des Hygieneartikels, gehalten durch eine zusätzliche Abdeckung (24), angebracht ist.

4. Hygieneartikel nach Anspruch 1, **dadurch gekennzeichnet, dass** der Faden im Randbereich der Längsseiten (16) und der Rückseite (18) in die Struktur des Hygieneartikels eingewoben ist.

5. Hygieneartikel nach Anspruch 1-4, **dadurch gekennzeichnet, dass** der Freilauf des Fadens im Hygieneartikel durch eine den Faden umschliessende Röhre (21) gewährleistet wird.

6. Hygieneartikel nach Anspruch 1-5, **dadurch gekennzeichnet, dass** der Faden auf der Rückseite (18) des Hygieneartikels an der Struktur des Hygieneartikels befestigt ist.

7. Hygieneartikel nach Anspruch 1-5, **dadurch gekennzeichnet, dass** der Faden auf der Rückseite nicht durchgehend geführt wird und die Enden des Fadens auf der Längsseite an der Struktur des Hygieneartikels befestigt sind.

8. Hygieneartikel nach Anspruch 1-7, **dadurch gekennzeichnet, dass** der Austrittspunkt des Fadens aus dem Hygieneartikel durch eine Verstärkung gesichert ist.

9. Hygieneartikel nach Anspruch 1-8, **dadurch gekennzeichnet, dass** der Faden auf der Vorderseite (17) des Hygieneartikels nach Austritt aus dem Hygieneartikel durchgehend ist und mit einem Klebstreifen zur Befestigung ausgestattet ist.

10. Hygieneartikel nach Anspruch 1-9, **dadurch gekennzeichnet, dass** der Faden im hinteren Teil des Hygieneartikels nach Austritt aus dem Hygieneartikel nicht durchgehend ist und mit Klebstreifen an den Fadenenden zur Befestigung ausgestattet ist.
